# EUROPEAN PATENT APPLICATION

(11) **EP 3 862 415 A1**
(43) Date of publication of application: **11.08.2021**
(21) Application number: 20155448.2
(22) Date of filing: 04.02.2020
(51) Int. Cl.: C12M 1/12, B01L 3/00, C12M 1/00, C12M 1/26, C12Q 1/04, C12Q 1/06

(54) **METHOD, DEVICE, SENSOR CARTRIDGE AND KIT OF PARTS FOR CULTURING AND DETECTING MICROORGANISMS**

(71) Applicant: Sensirion AG, 8712 Stäfa (CH)
(72) Inventor: Streiff, Matthias, 8712 Stäfa (CH); Huber, Deborah, 8712 Stäfa (CH); Baltensperger, Oliver, 8712 Stäfa (CH); Di Giacomo, Raffaele, 8712 Stäfa (CH)
(74) Representative: Schulz Junghans Patentanwälte PartGmbB

(57) **Abstract**

The invention relates to a method for culturing and detecting microorganisms, comprising the steps of providing a liquid sample (S) in a barrel (10) of a device (1) for culturing and detecting microorganisms, passing the liquid sample (S) through a first filtering membrane (40) such that microorganisms contained in the liquid sample (S) are retained at a first side (41) of the first filtering membrane (40), contacting said first side (41) with a first growth medium (210) capable of supporting growth of microorganisms, incubating the first filtering membrane (40) and the first growth medium (210) at an incubation temperature, arranging a sensing surface (51) of a gas sensor (50) in fluid connection with a second side (42) of the first filtering membrane (40), detecting a metabolic gas released by the microorganisms by means of the gas sensor (50).

The invention further relates to a device (1) for culturing and detecting microorganisms, comprising a barrel (10) enclosing a barrel compartment (13) for receiving a liquid sample (S), a first piston (20) which (20) is movable in said barrel (10), wherein said barrel compartment (13) is configured to be brought in fluid communication via a first filtering membrane (40) with a sensing surface (51) of a gas sensor (50) configured to detect a metabolic gas released by microorganisms, wherein the first filtering membrane (40) is configured to retain microorganisms contained in the liquid sample (S) at the first side (41) of the first filtering membrane (40).

Furthermore, a sensor cartridge (4) and a kit of parts comprising the device (1) are provided.

## Description

The invention relates to a method for culturing and detecting microorganisms, and a device, a sensor cartridge, and a kit of parts to perform the method according to the invention. In particular, the device may be a syringe. The method may be applied, for instance for detecting microorganisms in a urine sample of a patient suffering from a urinary tract infection and testing the susceptibility of the microorganisms to growth inhibitors, such as antibiotics. However, many other sample types and applications are conceivable within the scope of the invention.

When a microbial infection is suspected in a patient, patient samples, e.g. blood samples, stool samples, urine samples and the like, are usually cultured by standard laboratory techniques on nutrient agar plates which selectively support the growth of the microorganisms of interest to detect whether the microorganisms are present in the analyzed sample. Further tests, such as susceptibility and resistance to certain growth inhibitors, e.g. antibiotics, are then often performed to optimize patient treatment.

Standard microbiological procedures of the prior art have the disadvantage that it takes one to several days until the results are obtained. In contrast, treatment must often be initiated as quickly as possible to effectively combat microbial infections.

Furthermore, standard microbiological culturing methods require a sterile working environment and trained personnel to obtain accurate results.

Thus, a method for culturing and detecting microorganisms which is faster compared to the prior art, and which may be performed at the point of care without trained laboratory personnel, would be highly desirable.

Prior art document WO 2018/095529 provides a solution, where microorganisms are retained on a size-selective filtering membrane provided in a microfluidic channel, and the growth of the microorganisms is detected by a sensor, e.g. an impedance sensor.

However, the described method is relatively complex, especially in view of susceptibility measurements to growth inhibitors, such as antibiotics, which would be critical for point-of-care screening of patient samples.

The article *"*Zhu Y, Jovic, M, Lesch A, Lovey LT, Prudent M, Pick H, Girault HH "Immuno-affinity amperometric detection of bacterial infections", Angew Chem Int Ed Engl. 2018 Nov 5; 57(45): 14942-14946" describes detection of immuno-affinity enriched microorganisms by amperometry in several hours.

However, the method is still relatively complex for a point-of-care setting and requires chemical reagents as redox indicators of metabolic activity of microorganisms, which increases the costs of the method.

Therefore, the objective of the present invention is to further improve the methods for detecting and culturing microorganisms in view of the disadvantages of the prior art, particularly to improve usability by non-trained personnel, speed, and flexibility.

This objective is attained by the subject matter of the independent claims. Embodiments of the invention are specified in the sub-claims and described hereafter.

A first aspect of the invention relates to a method for culturing and detecting microorganisms, comprising the steps of providing a liquid sample in a barrel of a device for culturing and detecting microorganisms (e.g. a barrel of a syringe), passing the liquid sample through a first filtering membrane from a first side to a second side of the first filtering membrane, the second side being arranged opposite the first side, such that microorganisms contained in the liquid sample are retained at the first side of the first filtering membrane, contacting the first side of the first filtering membrane with a first growth medium capable of supporting growth of microorganisms, incubating the first filtering membrane and the first growth medium at an incubation temperature, arranging a sensing surface of a gas sensor in fluid connection with the second side of the first filtering membrane, and detecting a metabolic gas released by the microorganisms at the sensing surface by means of the gas sensor. In particular, the device for culturing and detecting microorganisms may be formed and/or configured according to the below described second aspect of the invention and its embodiments.

Providing the liquid sample in a barrel of a device for culturing and detecting microorganisms, which may be a barrel of a syringe, allows for simple collection of the microorganisms in the liquid sample on the filtering membrane without additional equipment such as a pump, e.g. by dispensing the sample through the filtering membrane using a piston or plunger which is movably arranged in the barrel. Moreover, the sample is directly contacted by a growth medium on the filtering membrane and growth is detected continuously, and particularly automatically, without removing samples, which reduces the risk of contamination. This improves usability compared to methods of the prior art, especially when the method is performed by untrained personnel (reducing costs) and at the point of care. In addition, the invention allows the tests / diagnostics to be carried out in significantly less time compared to methods of the prior art.

Furthermore, the method and the device according to the invention have the advantage that all microorganisms - and especially the cultured microorganisms in high concentrations - are kept inside the barrel. Therefore, as far as biohazards are concerned the device provides for a completely closed and therefore safe system. Because the gas sensor is separated from the microorganisms by the first filtering membrane, and connected only through a gaseous headspace, the gas sensor is not contaminated by the microorganisms, and therefore may be reused a certain amount of times (e.g. 10 times).

In the context of the present specification, the term 'microorganisms' designates single cell organisms, which may be prokaryotes (particularly bacteria), archaea or eukaryotes (e.g. unicellular fungi or protozoa).

The liquid sample is particularly dispensed, e.g., poured, manually or automatically pipetted, dripped or the like, into the barrel or aspirated into the barrel via a first end opening, more particularly a nozzle, of the barrel, more particularly the nozzle of a syringe.

The filtering membrane may be produced from a woven or non-woven material. In particular, the filtering membrane is size-selective and comprises pores of a size smaller than an average size of the microorganisms of interest, which are to be cultured and detected by the method. In particular, the pore size or average pore size or mesh size (in a woven filter) of the filtering membrane is 1 µm or less. Consequently, the filtering membrane is configured for sterile filtration of a suspension of the microorganisms of interest.

In certain embodiments, the liquid sample is passed through the first filtering membrane by applying a vacuum at the second side of the first filtering membrane or by applying a hydrostatic pressure at the first side of the first filtering membrane.

As used herein, the term 'barrel' designates an elongated structure with a central bore, which is configured to receive a piston in the bore in a manner such that the piston is movable relative to the barrel, such as e.g. in a syringe. In particular, the barrel of the device for culturing and detecting microorganisms extends along a longitudinal axis between a first end opening and a second end opening. The first end opening and the second end opening connect the barrel compartment which is enclosed by the barrel, to the exterior of the barrel.

The first growth medium may be any medium supporting the growth of microorganisms, i.e. providing the necessary nutrients for the microorganisms to grow. The first growth medium may be a liquid, a solid, a gelified liquid or a liquid adsorbed into a porous solid or semi-solid. Furthermore, the first growth medium may be a complex medium (e.g. Luria broth agar) or a minimal medium (i.e. containing defined amounts of chemically defined nutrients and salts).

In certain embodiment, the first growth medium is a solid, a gelified liquid or a liquid adsorbed into a porous solid or semi-solid. An example of a gelified liquid is a nutrient agar or nutrient agarose, as used in microbiology techniques of the prior art.

The term 'metabolic gas' as used in the present specification designates a gas which is released by microorganisms as a gaseous product of metabolic biochemical reactions, particularly during growth of the microorganisms. An example of a metabolic gas which may be released by heterotrophic microorganisms, is ethanol. However, many other such metabolic gases are known to the person skilled in the art of microbiology, and the scope of the present invention includes detection of these gases by the gas sensor.

In certain embodiments, the metabolic gas is an organic compound, wherein particularly the organic compound is selected from the group consisting of acetonitrile, ethanol, butanol, acetone, acetic acid, ethylene glycol, isopentanol, pyrimidine, 2-pentanone, 4-methylphenol, indole, 2-aminoacetophenone or 2-nonanone. The advantage of detecting organic compounds to follow microbial growth is that a very low background concentration of these compounds is usually found in ambient air, which improves the sensitivity of the measurement.

In certain embodiments, the metabolic gas is CO₂.

By detecting the metabolic gas, the metabolic activity of the microorganisms inside and on top of the filtering membrane and in touch with the first growth medium is monitored by the gas sensor, which is particularly arranged in a cavity outside of the filtering membrane, wherein measuring gaseous substances is indicative of microbial activity.

In certain embodiments, the gas sensor is a metal oxide semiconductor (MOX) gas sensor. In particular, the gas sensor comprises a sensing surface comprising metal oxides (e.g. a zinc oxide or a titanium oxide).

In certain embodiments, the gas sensor is configured to detect an organic compound, particularly acetonitrile, ethanol, butanol, acetone, acetic acid, ethylene glycol, isopentanol, pyrimidine, 2-pentanone, 4-methylphenol, indole, 2-aminoacetophenone or 2-nonanone. In case of a MOX gas sensor, such organic compounds may be catalytically converted by metal oxides at the sensing surface, which may give rise to an electric signal of the gas sensor. The incubation temperature may be chosen according to the microorganisms of interest. For instance, many bacterial species grow well at 37°C, whereas some yeasts are usually cultured at lower temperatures, e.g. 30°C. In case of thermophilic or hyperthermophilic organisms, incubation temperatures may be significantly higher, e.g. even up to values above 100°C. On the other hand, the cultivation of psychrophilic organisms may require low temperatures close to 0°C or even below.

In certain embodiments, the device for culturing and detecting microorganisms comprises a first piston comprising a first tip. In particular, the first piston is movable in the barrel of the device along the longitudinal axis of the barrel. The first piston as used in the present specification may also be termed 'outer piston'.

In particular, the first piston allows to process the sample, i.e. filter the liquid substrate and load the microorganisms onto the filtering membrane, and, particularly simultaneously, apply the growth substrates (e.g. in the form of solids, gelified liquids, and liquids adsorbed into porous solids or semi-solids, i.e. sponge-like structures) to the microorganisms on the filtering membrane. Thereby, the liquid sample can be applied and subjected to culturing and detection of microorganisms in a simple manner without premixing of the liquid sample with a growth substrate. In addition, the growth substrate concentration can be well controlled, e.g. by dry storage in the barrel compartment, resulting in a reliable operation. Finally, the first piston allows the application of standard microbiology growth substrates, such as CLED agar and MacConkey agar, as well as other gel-like and liquid substrates.

In certain embodiments, the first tip of the first piston comprises the first growth medium.

In certain embodiments, the first side of the first filtering membrane is contacted by the first growth medium by moving the first piston in the barrel to a first position along a longitudinal axis of the barrel. In the first position, the first tip of the first piston contacts the first side of the filtering membrane.

In certain embodiments, the first growth medium is a solid, a gelified liquid or a liquid adsorbed into a porous solid or semi-solid.

In certain embodiments, the liquid sample is passed through the first filtering membrane by applying a hydrostatic pressure at the first side of the first filtering membrane by moving the first piston along the longitudinal axis in the barrel, particularly to the first position. In this case, the first growth medium contacts the first side of the first filtering membrane when the step of passing the liquid sample through the first filtering membrane is finished.

Alternatively, particularly if the liquid sample is passed through the first filtering membrane by applying a vacuum at the second side of the first filtering membrane, the first growth medium may be contacted by the first growth medium by moving the first piston to the first position in a step subsequently to passing the liquid sample through the first filtering membrane.

In certain embodiments, the first growth medium is a solid, a gelified liquid or a liquid adsorbed into a porous solid or semi-solid, wherein the device comprises a first piston comprising a first tip, and wherein the first tip of the first piston comprises the first growth medium, wherein the first side of the first filtering membrane is contacted by said first growth medium by moving the first piston in the barrel to a first position along a longitudinal axis of the barrel.

An example of a gelified liquid is a nutrient agar, such as, e.g. CLED agar or MacConkey agar.

In certain embodiments, the liquid sample is aspirated into the barrel by moving the first piston along the longitudinal axis away from the first filtering membrane, i.e. in the direction from the first end opening towards the second end opening.

In certain embodiments, the first tip of the first piston comprises a first partial area and a second partial area. In certain embodiments, the first partial area and the second partial area extend perpendicular to the longitudinal axis. In certain embodiments, the first partial area and the second partial area are non-overlapping. In certain embodiments, the first growth medium is arranged in or on the first partial area, wherein a second growth medium is arranged in or on the second partial area.

In particular, the second growth medium is different from the first growth medium, more particularly wherein the first growth medium promotes the growth of a first group of microorganisms and the second growth medium promotes the growth of a second group of microorganisms different from the first group of microorganisms. For example, the first growth medium may contain a higher amount of nutrients than the second growth medium, or the second growth medium may contain certain nutrients allowing the growth of certain microorganisms, wherein these nutrients are not contained in the first growth medium. It is also conceivable that the first growth medium contains a certain growth inhibitor, such as an antibiotic, whereas the second growth medium contains no growth inhibitor or a different growth inhibitor.

For example, half of the first tip may be covered by CLED (Cystine Lactose Electrolyte Deficient) agar, and the other half of the first piston may be covered by MacConkey agar.

Such a multipart growth substrate has the advantage that a set of microorganisms with different growth substrate requirements may be cultivated concurrently.

In certain embodiments, the first tip of the first piston further comprises a third partial area, the third partial area extending perpendicular to the longitudinal axis, wherein the third partial area is non-overlapping with the first partial area and the second partial area, and wherein a third growth medium is arranged on the third partial area. In particular, the third growth medium is different from the first growth medium and/or the second growth medium, more particularly wherein the third growth medium promotes the growth of a third group of microorganisms different from the first group and the second group of microorganisms.

In certain embodiments, the first tip of the first piston further comprises a further partial area, the further partial area extending perpendicular to the longitudinal axis, wherein the further partial area is non-overlapping with the first partial area, the second partial area and the third partial area, and wherein a further growth medium is arranged on the further partial area.

It should be noted that all embodiments described in this specification with reference to the first growth medium may equally be applied to the second growth medium and any additional further growth medium.

In certain embodiments, the first growth medium is a liquid comprised in the liquid sample.

According to a first alternative, the first side of the first filtering membrane is contacted by the liquid first growth medium upon providing the liquid sample in the barrel.

According to a second alternative, the first side of the first filtering membrane is contacted by the liquid first growth medium by dispensing the liquid sample through the first filtering membrane. In particular, the liquid sample is dispensed through the first filtering membrane by moving the first piston in the barrel along the longitudinal axis towards the filtering membrane to a second position, wherein the first tip of the first piston is spaced apart from the first filtering membrane in the second position, such that a volume of the liquid sample comprising the liquid first growth medium remains in the barrel and contacts the first side of the first filtering membrane.

In certain embodiments, the first growth medium is a liquid, wherein the first piston is moved in the barrel along the longitudinal axis to a second position, wherein the first tip of the first piston is spaced apart from the first filtering membrane in the second position.

In certain embodiments, the liquid growth medium contacts the first side of the first filtering membrane in the second position, particularly wherein the first growth medium is comprised in the liquid sample. In particular, the first tip of the first piston may comprise additives capable of enhancing the growth of the microorganisms contained in the liquid sample.

If not all of the liquid sample is filtered and a specific amount of the liquid sample is retained in the barrel, the natural growth environment of the microorganisms under investigation may be preserved in some cases. The primary function of the first piston may then be to close the barrel compartment from the second end opening in an airtight manner. As mentioned, the first tip of the first piston may optionally contain additives to enhance the growth of the microorganisms in the liquid first growth medium.

In certain embodiments, the first growth medium is contacted by a growth inhibitor, e.g. an antibiotic, capable of inhibiting growth of microorganisms or a growth promotor capable of promoting growth of microorganisms.

During contacting the first growth medium with the growth inhibitor (e.g. by contacting the surface of the growth medium with the second tip of the second piston), a groove may be formed in a surface of the first growth medium opposite the first side of the first filtering membrane.

Therein, the term 'growth inhibitor' designates any substance capable of killing microorganisms, i.e. permanently destroying the microorganisms, any substance capable of preventing the growth of microorganisms, e.g. by inhibiting cell division, and/or any substance capable of slowing the growth rate of microorganisms. In particular, the growth inhibitor is an antibiotic. These effects may relate to all microorganisms or certain specific groups of microorganisms, such as bacteria or certain sub-types or species of bacteria.

The term 'growth promotor' designates any substance capable of supporting the growth of microorganisms or increasing the growth rate of microorganisms (relating to all microorganisms or specific types of microorganisms).

Within the context of the present specification, growth inhibitors and growth promotors may be designated collectively as 'probe substances'.

In certain embodiments, the growth inhibitor or the growth promotor is comprised in or on a second tip of a second piston of the device. The second piston as used herein may also be termed 'inner piston'.

In certain embodiments, the second piston, particularly the second tip, comprises a disc from a porous material (e.g. a diffusion disc) arranged at the second tip, wherein the growth inhibitor or the growth promotor is comprised in the porous material, such that the growth inhibitor or the growth promotor diffuses into the first growth medium (or second growth medium or further growth medium) from the disc when the disc contacts a surface of the first growth medium (or second growth medium or further growth medium).

The inner piston allows the addition of probe substances to the growth medium with minimal disturbance of the gas compartment during culturing, and allows addition of the probe substances in a variety of forms, such as solids, gelified liquids, and liquids adsorbed into porous solids or semi-solids (i.e. sponge-like structures). The probe substances can be applied in standard microbiology forms such as solid diffusion disks, but at the same time in a variety of other forms. One or more probe substances can be applied sequentially at any time during the culturing step with minimal disturbance of the culture volume.

In certain embodiments, the first growth medium is contacted by the growth inhibitor or the growth promotor by moving the second piston along the longitudinal axis to a third position in a bore of the first piston. In particular, the second tip of the second piston contacts the first growth medium in the third position.

In particular, the surface of the first growth medium opposite the first side of the first filtering membrane is contacted by the growth inhibitor or the growth promotor by moving the second piston along the longitudinal axis to a third position in a bore of the first piston.

In certain embodiments, the second piston is moved along the longitudinal axis to the third position relative to the first piston.

In certain embodiments, the second piston is moved towards the first side of the first filtering membrane in the barrel (which is in the direction of the first end opening) and brought into contact with the first growth medium. In other words, one or more inner pistons are applied to the outer piston.

Alternatively, in certain embodiments, the second piston is moved away from the first side of the first filtering membrane in the barrel (that is in the direction towards the second end opening), i.e. removed from the first growth medium. In other words, one or more inner piston is removed from the outer piston. In this case, the tip of the inner piston does not have to contact the first growth medium in the third position (final position of the tip of the inner piston).

In certain embodiments, the device for culturing and detecting microorganisms may comprise a plurality of second pistons in the bore of the first piston.

In certain embodiments, the first piston comprises a plurality of bores extending along the longitudinal axis, and the device for culturing and detecting microorganisms comprises a plurality of second pistons, wherein each second piston is arranged in a respective bore of the first piston, wherein the respective second piston is movable along the longitudinal axis in the respective bore.

In certain embodiments, the second piston comprises a bore extending along the longitudinal axis, and the device for culturing and detecting microorganisms comprises a third piston arranged in the bore of the second piston, wherein the third piston is movable along the longitudinal axis in the bore of the second piston.

In particular, the second piston or the second pistons is/are activated by pushing or pulling a rod or a pin.

In certain embodiments, the growth inhibitor or the growth promotor is added to the first growth medium in liquid form, e.g. by dispensing, ink jetting or by a porous solid or semi-solid soaked with the liquid growth inhibitor or growth promotor. In case of dispensing or ink jetting, this is an alternative to providing a solid or semi-solid on the tip of the inner piston.

Providing the probe substance on the second tip of the second piston allows to introduce the probe substance with minimal disturbance of the closed head-space in which the gas sensor is present.

In addition, the separation of growth substrates and probe substances has the further advantages that these parts can be combined flexibly at the time of use - i.e. a certain set of growth substrates with a certain set of probe substances - and stored separately. The ability to flexibly combine the two parts is an advantage as less manufacturing variants of the product are needed to cover the same amount of combinations, and the possibility of separate storage is an advantage as, typically growth substrates (e.g. agars) and probe substances (e.g. antibiotics) have different storage conditions and shelf lives.

In particular, the growth inhibitor or the growth promotor is dispensed in liquid form onto the surface of the first growth medium opposite the first side of the first filtering membrane. The liquid probe substance may be added to the first growth medium from a reservoir in the second piston or from another source.

In certain embodiments, the first growth medium is contacted by the growth inhibitor or the growth promotor repeatedly. Therein, in particular, the first growth medium is sequentially contacted by a plurality of second pistons comprising increasing or decreasing concentrations of a growth inhibitor or a growth promotor.

In other words, in particular, the probe substance in high concentration on an inner piston can be brought into contact only briefly but multiple times with the growth substrate to increase the concentration of the probe substance gradually in a 'bolus by bolus' manner. Between contacting, sufficient time is left to the probe substance to achieve a homogeneous concentration distribution within the growth substrate.

Alternatively, in particular, the same probe substance may also be applied by a set of inner pistons with increasing concentrations of the same probe substance to increase the amount of antibiotic present in the growth substrate gradually. This mode is especially useful, if the thickness of the growth substrate is small and the equilibrium of the concentration of probe substance in the inner piston and the growth substrate is reached quickly.

In certain embodiments, first growth medium is contacted by the growth inhibitor or the growth promotor before incubation, i.e. at the start of the experiment.

According to an alternative embodiment, the first growth medium is contacted by the growth inhibitor or the growth promotor during incubation, particularly dependent on a signal of the gas sensor indicating metabolic activity of the microorganisms.

In the latter case, e.g. a probe substance in the form of an antibiotic can be applied to challenge the microorganisms. Depending on how the metabolic signal is affected, susceptibility or resistance of the microorganism can be inferred. Different probe substances may also be applied in sequence to challenge the same microbial culture.

In certain embodiments, a minimal inhibitory concentration of the growth inhibitor is determined by the time lag between contacting the first growth medium with the growth inhibitor and a response of the signal of the gas sensor. Therein, the term 'minimal inhibitory concentration' designates the lowest concentration of the growth inhibitor which inhibits visible growth of microorganisms. The relationship between the measured signal of the gas sensor and the colony forming units may be determined in a calibration experiment.

As described, the time lag between the application of the probe substance and the reaction of the metabolic signal may be used as a measure for the minimum inhibitory concentration. This is, because the concentration of the antibiotic at the location of the microorganisms raises gradually upon application of the probe substance by diffusion perpendicular to the growth substrate, until the equilibrium concentration is reached. The time constant of this process is governed by the diffusion constant of the antibiotic in the growth substrate and the thickness of the latter.

In certain embodiments, the liquid sample is provided by homogenization and/or liquification of a sample, wherein particularly the sample is a solid sample or a pastelike sample.

In certain embodiments, the liquid sample is diluted, e.g. by addition of a suitable buffer solution, such as phosphate buffered saline, PBS. In this manner, the viscosity of the sample can be reduced, facilitating filtration.

In certain embodiments, the liquid sample is incubated with beads carrying an affinity molecule on their surface, the affinity molecule being capable of specifically binding to microorganisms, wherein a suspension of the beads that have been incubated with the liquid sample is provided in the barrel of the device for culturing and detecting microorganisms. The liquid sample comprising the beads is then passed through the first filtering membrane, such that the beads with the bound microorganisms are retained at the first side of the first filtering membrane. The liquid sample is incubated with the bead under conditions allowing specific binding of the microorganisms to the affinity molecules. This may require incubation at a specific pH and/or at a specific temperature (particularly 1°C to 90°C) as well as agitation, e.g. stirring and/or certain buffers or additives. Depending on the affinity molecules used and their binding kinetics to the target molecules, the incubation may take minutes to hours.

The term 'beads' as used herein refers to particles from any suitable material to bind affinity molecules. In particular, the beads have an essentially spherical shape. In particular, the beads are nanobeads having a diameter from 1 nm to 100 nm or the beads are microbeads having a diameter of 100 nm to 100 µm.

An 'affinity molecule' is defined herein as a molecule capable of specifically binding to a cell surface structure (i.e. cell surface molecule) of a microorganism. Examples of affinity molecules include antibodies, aptamers, certain nucleic acids, or molecularly imprinted polymers. In case antibodies are used as affinity molecules, the described pre-processing method may also be referred to as 'immuno-precipitation'.

When the microorganisms are bound to beads as described above, the first filtering membrane may have a pore size of substantially more than 1 µm, since microorganisms are bound to the beads which are significantly larger than the average microorganisms themselves.

In certain embodiments, the beads are precipitated and resuspended (after removing the supernatant), particularly in a buffer solution, prior to providing the beads in the barrel of the device. In particular, the beads are washed, particularly in a buffer solution (e.g. phosphate buffered saline, PBS), at least once before providing the beads in the barrel of the device. Therein the term 'wash' or 'washing' refers to resuspending the precipitated beads, precipitating the beads again, removing the supernatant, and resuspending the beads again in fresh buffer. For example, precipitation of the beads may generally be performed by centrifugation, and resuspension may be performed by agitating or pipetting.

In certain embodiments, the beads are magnetic beads (in other words the bead comprise a magnetic material), and the beads are precipitated by applying a magnetic force. Such beads can be precipitated, e.g. on an inner surface of a vial, by arranging a magnet adjacent to the vial.

In certain embodiments, the first filtering membrane comprises or consists of a magnetic material providing the magnetic force, wherein particularly the first filtering membrane comprises magnetic particles embedded in the first filtering membrane, e.g. in a polymer sheet or thread.

In certain embodiments, the liquid sample is incubated with magnetic beads carrying an affinity molecule on their surface, the affinity molecule being capable of specifically binding to microorganisms, wherein a suspension of the magnetic beads that have been incubated with the liquid sample is provided in the barrel of the device for culturing and detecting microorganisms, wherein the first filtering membrane comprises or consists of a magnetic material providing a magnetic force, and wherein the magnetic beads are precipitated on the first side of the first filtering membrane by the magnetic force.

This embodiment facilitates cultivation and detection, especially of small amounts of microorganism, since due to the magnetic filter structure, previous collection, resuspension and washing steps are made obsolete.

In case of a magnetic first filtering membrane, the pore size of the first filtering membrane may be not only larger than the average size of microorganisms to be collect, but may also be significantly larger than the average size of the magnetic beads, since the beads are bound to the first filtering membrane due to their magnetic properties. In particular, the pore size of the first filtering membrane may be 10 µm to 1 mm in this specific case. Of course, the first filtering membrane may alternatively have a pore size smaller than the average diameter of the beads (e.g. ≤1 µm), so that size selection is performed in addition to selection by magnetic properties.

The described pre-processing methods (liquidization, homogenization, dilution, precipitation using magnetic or non-magnetic beads or the like) are especially advantageous to improve the quality of complex samples, such as blood samples, paste-like samples such as stool samples or solid samples, such as tissue samples, for subsequent application of the method according to the invention.

In certain embodiments, the sensing surface of the gas sensor is arranged in a sensor compartment which is in fluid connection with the second side of the first filtering membrane. In other words, after mounting the gas sensor on the device, the gas sensor and the outside of the filtering membrane share a closed head-space.

In certain embodiments, the sensor compartment comprises an inlet and an outlet.

In certain embodiments, the inlet comprises a first valve allowing gas flow into the sensor compartment, and the outlet comprises a second valve allowing gas flow out of the sensor compartment.

In certain embodiments, the gas sensor is calibrated by flushing the sensor compartment with a flushing gas by providing a flow of a calibrant gas through the sensor compartment between the inlet and the outlet.

In certain embodiments, the gas sensor, particularly the sensor compartment of the gas sensor, is flushed, particularly continuously, with a flushing gas, particularly in an idle state of the gas sensor.

In certain embodiments, the flushing gas is purified air, particularly zero air, or a mixture of N₂ and O₂, from which organic compounds have been removed. In particular, the term 'zero air' as used herein refers to air with a concentration of 0,05 ppm or less of organic compounds.

In certain embodiments, the flushing gas has a temperature of 37°C.

In certain embodiments, the flushing gas has a relative humidity of 95% or more.

In certain embodiments, the gas sensor is flushed with the flushing gas before incubation of the device for culturing microorganisms.

In certain embodiments, the gas sensor is flushed with the flushing gas during incubation of the device for culturing microorganisms. In particular, this function may be used during the microbial growth phase in case the measuring process has to be reset.

The flushing function of the gas sensor also has the effect that the gas sensor can be reused, because its function can be verified and it can be re-calibrated. This makes the solution lower cost. In addition, the sensitivity of the gas sensor can be improved because a clean baseline can be measured before the microorganisms are added.

In certain embodiments, the sensor compartment is separated from the second side of the first filtering membrane by a second filtering membrane.

In certain embodiments, the gas sensor is provided in a sensor cartridge which is removably connected to the first end opening of the barrel of the device. This has the advantage that the gas sensor is reusable.

In certain embodiments, the liquid sample is aspirated into the barrel compartment through an aspiration opening of an aspiration cap which is removably connected to the first end opening of the barrel.

In certain embodiments, the first filtering membrane is comprised in a filtering cap which is removably connected to the first end opening of the barrel.

In certain embodiments, the gas sensor is heated during incubation of the device for culturing and detecting microorganisms, such that the resulting temperature is slightly elevated over the temperature of the remaining device. In this manner, condensation of liquid at the sensor, particularly the sensing surface, can be prevented, thus avoiding a disturbance in the signal of the gas sensor. Heating may be performed by a heating element which is integrated into the device or by a separate heating element.

In certain embodiments, the gas sensor, particularly the sensor compartment of the gas sensor is arranged vertically above the barrel and the first filtering membrane of the device. This eliminates contamination of the gas sensor by liquids from other components of the device.

In certain embodiments, the gas sensor is disabled when concentration of metabolic gases detected by the gas sensor rises above a pre-specified threshold. This avoids damage to the sensing surface of the gas sensor due to high gas concentrations
In certain embodiments, the microorganisms in the liquid sample are labelled with an optically detectable label, e.g. a fluorescence dye or an enzyme capable of catalyzing a chemical reaction of a substrate resulting in a colored product, wherein the optically detectable label is linked to an affinity molecule or linked to a bead carrying an affinity molecule, wherein the affinity molecule specifically binds to the microorganisms, and wherein the optically detectable label is detected, and wherein the microorganisms are sorted by means of the optically detectable label following removal from the device, i.e. from the first side of the filtering membrane and/or the first growth medium.

In certain embodiments, the microorganisms are detected by determining an electrical impedance in the first growth medium, particularly by providing an alternating voltage in the first growth medium between a first electrode and a second electrode, and measuring a capacitance of the first growth medium. Therein, the measured impedance signal may be a result of the presence of the microorganisms and/or a result of micro or nano beads with a characteristic impedance pattern, which may be bound to a specific microorganism by an affinity molecule.

The components of the device for culturing and detecting microorganisms, particularly the filtering membrane, the barrel, the first piston, the second piston and the gas sensor, may be sterilized by methods known in the art of microbiology prior to performing the method according to the invention.

A second aspect of the invention relates to a device for culturing and detecting microorganisms, particularly by the method according to the first aspect, wherein the device comprises a barrel extending along a longitudinal axis between a first end opening and a second end opening, the barrel enclosing a barrel compartment for receiving a liquid sample.

All embodiments, features and advantages described above in relation to the method according to the first aspect of the invention may be applied also to the device according to the second aspect.

In certain embodiments, the device comprises a first piston comprising a first tip, wherein the first piston is movable in the barrel along the longitudinal axis of the barrel.

In certain embodiments, the barrel compartment is configured to be brought in fluid communication via a first filtering membrane with a sensing surface of a gas sensor configured to detect a metabolic gas released by microorganisms, wherein the first filtering membrane comprises a first side and a second side opposite the first side, wherein the first side of the first filtering membrane faces the barrel compartment and the second side of the first filtering membrane faces the sensing surface, and wherein the first filtering membrane is configured to retain microorganisms contained in the liquid sample at the first side of the first filtering membrane.

In particular, the first filtering membrane has a pore size ≤ 1 µm.

In certain embodiments, the first piston comprises a sealing surface extending in a circumferential direction relative to the longitudinal axis, wherein the sealing surface is in contact with an inner wall of the barrel, such that an interface of the sealing surface and the inner wall is sealed preventing liquid and gas from passing the interface. Particularly, the first piston closes the second end opening. In this manner, liquid may be aspirated through the first end opening of the barrel of the device by moving the first piston along the longitudinal axis from the first end opening towards the second end opening, and liquid may be dispensed through the first end opening, and particularly through the first filtering membrane by moving the first piston along the longitudinal axis from the second end opening towards the first end opening.

In certain embodiments, the device comprises the first filtering membrane. In other words, the device comprises a first filtering membrane comprising a first side and a second side opposite the first side, wherein the first side of the first filtering membrane faces the barrel compartment, and wherein the first filtering membrane is configured to retain microorganisms contained in the liquid sample at the first side of the first filtering membrane.

In certain embodiments, the device comprises the gas sensor. In other words, the device comprises a gas sensor comprising a sensing surface configured to detect a metabolic gas released by microorganisms.

In certain embodiments, the device comprises a first filtering membrane comprising a first side and a second side opposite the first side, wherein the first side of the first filtering membrane faces the barrel compartment, and wherein the first filtering membrane is configured to retain microorganisms contained in the liquid sample at the first side of the first filtering membrane, and the device comprises a gas sensor comprising a sensing surface configured to detect a metabolic gas released by microorganisms, wherein the barrel compartment is in fluid communication via the first filtering membrane with the sensing surface of the gas sensor, and wherein the second side of the first filtering membrane faces the sensing surface.

In certain embodiments, the first tip of the first piston comprises or is configured to receive a first growth medium capable of supporting growth of microorganisms, wherein the first piston is movable along the longitudinal axis in the barrel to a first position, in which the growth medium contacts the first side of the first filtering membrane.

In certain embodiments, the first piston comprises an additive, particularly comprised in the first growth medium, the additive being capable of enhancing the growth of the microorganisms (meaning the microorganisms grow faster with the additive than without the additive).

In certain embodiments, the first tip of the first piston comprises a first partial area and a second partial area, the first partial area and the second partial area extending perpendicular to the longitudinal axis, wherein the first partial area and the second partial area are non-overlapping, wherein the first growth medium is comprised in or on the first partial area, and wherein a second growth medium is comprised in or on the second partial area.

In certain embodiments, the second growth medium is different from the first growth medium, particularly wherein the first growth medium promotes the growth of a first group of microorganisms and the second growth medium promotes the growth of a second group of microorganisms different from the first group.

In certain embodiments, the first tip of the first piston further comprises a third partial area extending perpendicular to the longitudinal axis, wherein the third partial area is non-overlapping with the first partial area and the second partial area, and wherein a third growth medium is comprised in or on the third partial area.

In certain embodiments, the first tip of the first piston further comprises a further partial area extending perpendicular to the longitudinal axis, wherein the further partial area is non-overlapping with the first partial area, the second partial area and the third partial area, and wherein a further growth medium is comprised in or on the further partial area.

In certain embodiments, the device comprises at least one second piston comprising a second tip, wherein the second piston is movable along the longitudinal axis in the barrel, particularly in a bore of the first piston, to a third position, wherein particularly the second tip comprises or is configured to comprise a growth inhibitor capable of inhibiting growth of microorganisms or a growth promotor capable of promoting growth of microorganisms.

In particular, the second piston is configured to be moved along the longitudinal axis in the barrel relative to the first piston.

In certain embodiments, the second tip comprises the growth inhibitor or the growth promotor, wherein particularly the growth inhibitor or the growth promotor consist of or is comprised in a solid, a gelified liquid or a liquid adsorbed into a porous solid or semi-solid.

In certain embodiments, the first filtering membrane comprises or consists of a magnetic material providing a magnetic force, such that magnetic beads comprised in the liquid sample can be precipitated on the first side of the first filtering membrane by the magnetic force. In particular, the first filtering membrane comprises magnetic particles embedded in the first filtering membrane.

In certain embodiments, the device comprises a sensor compartment which is in fluid connection with the second side of the first filtering membrane, wherein the sensing surface of the gas sensor is arranged in the sensor compartment.

In certain embodiments, the sensor compartment comprises an inlet and an outlet.

In certain embodiments, the inlet comprises a first valve allowing gas flow into the sensor compartment, and the outlet comprises a second valve allowing gas flow out of the sensor compartment.

In certain embodiments, the device comprises a second filtering membrane separating the sensor compartment from the second side of the first filtering membrane.

In certain embodiments, the gas sensor is comprised in a sensor cartridge which is removably connected to the first end opening of the barrel of the device.

In certain embodiments, the device comprises a heating element for heating the gas sensor, particularly to a temperature which is slightly elevated (e.g. by between 0,5°C and 1,0°C) over the temperature of the remaining device. In this manner, condensation of liquid at the sensor, particularly the sensing surface, can be prevented, thus avoiding a disturbance in the signal of the gas sensor.

A third aspect of the invention relates to an aspiration cap extending along a longitudinal axis between a connection opening and an aspiration opening the connection opening, wherein the aspiration cap is configured to be removably connected to the first end opening of the barrel of the device according to the second aspect of the invention, such that a liquid sample can be aspirated through the aspiration opening into the barrel compartment of the device when the first piston of the device is moved along the longitudinal axis in the direction from the first end opening towards the second end opening.

In particular, the connection opening and the aspiration opening face in opposite directions parallel to the longitudinal direction.

A fourth aspect of the invention relates to a filtering cap extending along a longitudinal axis between a connection opening and a dispensing opening, wherein the filtering cap comprises a first filtering membrane, particularly having a pore size ≤ 1 µm, comprising a first side facing the connection opening and a second side opposite the first side, the second side facing the dispensing opening, wherein the filtering cap is configured to be removably connected to the first end opening of the barrel of the device according to the second aspect, such that a liquid sample can be passed through the first filtering membrane from the first side to the second side of the first filtering membrane to retain microorganisms contained in the liquid sample at the first side of the first filtering membrane, wherein the dispensing opening of the filtering cap is configured to be brought in fluid communication with a sensing surface of a gas sensor configured to detect a metabolic gas released by microorganisms.

In particular, the connection opening and the dispensing opening face in opposite directions parallel to the longitudinal direction.

In certain embodiments, the first filtering membrane comprises or consists of a magnetic material, wherein particularly the first filtering membrane comprises magnetic particles embedded in the first filtering membrane.

A fifth aspect of the invention relates to a sensor cartridge comprising a gas sensor comprising a sensing surface configured to detect a metabolic gas released by microorganisms, wherein the sensor cartridge comprises a connection opening configured to be connected to the first end opening of the barrel of the device according to the second aspect or configured to be connected to the connection opening of the filtering cap according to the fourth aspect.

In certain embodiments, the sensor cartridge comprises a sensor compartment which is configured to be brought in fluid connection with the second side of the first filtering membrane, wherein the sensing surface of the gas sensor is arranged in the sensor compartment.

In certain embodiments, the sensor compartment comprises an inlet and an outlet.

In certain embodiments, the inlet comprises a first valve allowing gas flow into the sensor compartment, and the outlet comprises a second valve allowing gas flow out of the sensor compartment.

In certain embodiments, the sensor cartridge comprises a second filtering membrane configured to separate the sensor compartment from the second side of the first filtering membrane.

A sixth aspect of the invention relates to a kit of parts comprising the device according to the second aspect, the filtering cap according to the fourth aspect and the sensor cartridge according to the fifth aspect.

In certain embodiments, the kit of parts further comprises the aspiration cap according to the third aspect.

A seventh aspect of the invention relates to an apparatus comprising a plurality of devices according to the second aspect, particularly arranged in a two-dimensional array.

Examples of the present invention are now explained by reference to the attached drawings, from which additional embodiments may be drawn. The following description is meant to elucidate the invention without limiting its scope.
- Fig. 1: shows a schematic of an embodiment of the device for culturing and detecting microorganisms according to the invention;
- Fig. 2: shows a detail of the device according to Fig. 1, where the second piston is in the third position;
- Fig. 3: shows a detail of the first tip of the first piston of the device for culturing and detecting microorganisms according to an embodiment of the invention;
- Fig. 4: shows a cross-sectional view of an embodiment of the device for culturing and detecting microorganisms according to the invention;
- Fig. 5: shows an aspiration cap (A), a filtering cap (B) and a device for culturing and detecting microorganisms (C) according to the invention, which constitute components of a kit of parts according to the invention;
- Fig. 6: shows exemplary steps of the method according to an embodiment of the invention, wherein (A) depicts an aspiration step, (B) depicts a filtering step, and (C) depicts an incubation and detection step;
- Fig. 7: shows an embodiment of a device for culturing and detecting microorganisms according to the invention comprising a removable sensor cartridge, wherein (A) shows the sensor cartridge, (B) shows the barrel of the device, and (C) shows the assembled device with the sensor cartridge connected to the barrel;
- Fig. 8-9: show a perspective view (Fig. 8) and a cross-sectional view (Fig. 9) of an apparatus comprising a plurality of devices for culturing and detecting microorganisms according to the invention;
- Fig. 10-11: show data representing the growth of microorganisms measured with a device for culturing and detecting microorganisms according to the invention.

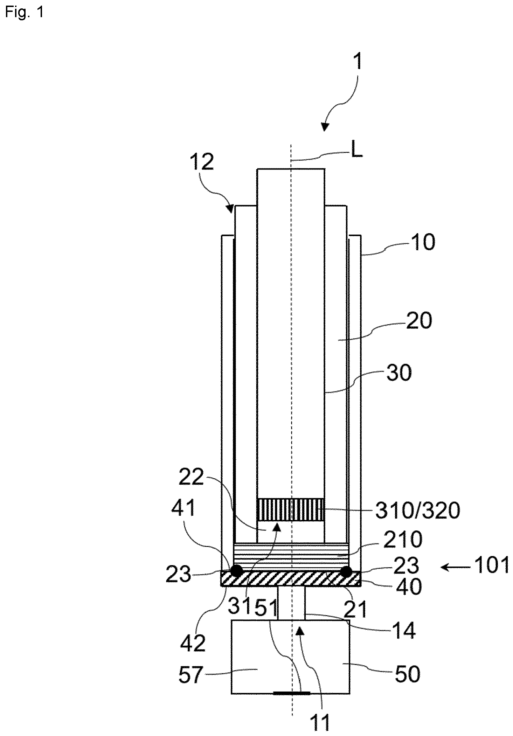

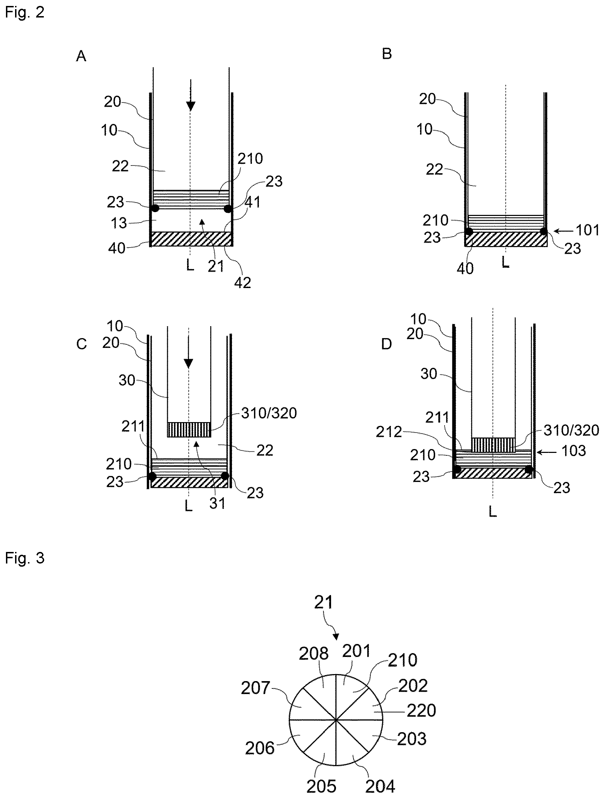

Fig. 1 shows in a schematic cross-sectional view, a device 1 for culturing and detecting microorganisms comprising a barrel 10 for receiving a liquid sample S, wherein the barrel 10 extends along a longitudinal axis L between a first end opening 11 and a second end opening 12 disposed opposite of the first end opening 11. The barrel 10 comprises a peripheral wall delimiting a barrel compartment 13 (not shown in Fig. 1, see Fig. 2A), wherein the first end opening 11 and the second end opening 12 connect the barrel compartment 13 to the exterior of the device 1. The device 1 has a cylindrical shape with the longitudinal axis L being the central cylinder axis. The first end opening 11 is comprised in a nozzle 14, and the second end opening 12 is closed by a first piston 20 which is inserted into the barrel compartment 13. The device 1 further comprises a filtering membrane 40, which extends perpendicular to the longitudinal axis L. The filtering membrane 40 comprises a first side 41 facing the barrel compartment 13 and a second side 42 opposite the first side 41 (see also Fig. 2A for the arrangement of the first side 41 and the second side 42 relative to the barrel compartment 13) and facing the first end opening 11 at the end of the nozzle 14. The filtering membrane 40 is from a woven or non-woven material having a pore size (e.g. an average pore size) of 1 µm or less, such that most microorganisms are unable to pass the filtering membrane 40 between the first side 41 and the second side 42 and vice versa.

The device 1 further comprises a first piston 20 (also termed outer piston herein) which is movably arranged along the longitudinal axis L in the barrel compartment 13. The first piston 20 comprises a first tip 21. Furthermore, the first piston 20 may comprise a sealing ring 23 adjacent to the first tip 21, the first sealing ring 23 being in contact with an inner wall of the barrel 10, such that the interface between the first sealing ring 23 and the barrel 10 is liquid and/or gas tight. In this case the first piston 20 may be used to aspirate liquid from the first end opening 11 by moving, e.g. pulling, the first piston 20 towards the second end opening 12, and dispense liquid from the first end opening 11 by moving, e.g. pushing, the first piston 20 towards the first end opening 11.

The first tip 21 of the first piston 20 comprises a first growth medium 210, on which microorganisms of interest, which are to be cultured and detected by the device 1, can grow. In other words, the first medium 210 is a solid, a gelified liquid or a liquid adsorbed into a porous solid or semi-solid that is attached to the first tip 21. For example, the first growth medium 210 may be a nutrient agar pad which coats the first tip 21 or is otherwise attached to the first tip 21. In one embodiment, the first tip 21 may comprise a recess, into which heated nutrient agar is poured or dispensed and solidified by cooling, after which the agar pad stays attached to the bottom and side walls of the recess. The first piston 20 and the first growth medium 210 are configured and arranged in a manner such that the first growth medium 210 contacts the first side 41 of the filtering membrane 40 in a first position 101 (as depicted in Fig. 1). That means that the first piston 20 has an appropriate length, such that the first tip 21 is able to touch the filtering membrane 40 in the first position 101, and the first growth medium 210 protrudes from the first tip 21 in the direction along the longitudinal axis L. In particular, the first piston 20 comprises a bore 22 or a plurality of bores 22 (in case a plurality of second pistons 30 is to be used) extending along the longitudinal axis L for receiving a second piston 30 or a plurality of second pistons 30.

Fig. 1 shows an embodiment, where one second piston 30 comprising a second tip 31 is movably arranged along the longitudinal axis L relative to the first piston 20 in the bore 22 of the first piston 20. Alternatively, a plurality of second pistons 30 comprising respective second tips 31 may be arranged in the bore 22 of the first piston 30 (not shown). These may be movable along the longitudinal axis L in the bore independently of each other or together.

Fig 2A-B illustrates the motion of the first piston 20 in the barrel compartment 13 of the barrel 10, and Fig. 2C-D show the motion of the second piston 30 in the bore 22 of the first piston 20 during steps of the method according to the invention.

The first piston 20 is moved downwards (indicated by the arrow in Fig. 2A) from the position above the first filtering membrane 40 shown in Fig. 2A to the first position 101 shown in Fig. 2B, where the first growth medium 210 at the first tip 21 of the first piston 20 contacts the first side 41 of the first filtering membrane 40. In this manner, microorganisms on the first side 41 of the first filtering membrane 40 are contacted by the first growth medium 210, such that they can grow.

Subsequently, as shown in Fig. 2C and 2D, a second piston 30 is moved downwards (indicated by the arrow in Fig. 2C, such that a second tip 31 of the second piston 30 contacts a surface 211 (opposite the first filtering membrane 40) of the growth medium 210 from inside the bore 22 of the first piston 20 (third position 103 in Fig. 2D). Thereby, a growth inhibitor 310 or a growth promotor 320 disposed at the second tip 31 contacts the first growth medium 210, such that the growth inhibitor 310 or growth promotor 320 can diffuse into the growth medium 210 to inhibit or promote growth of the microorganisms. The growth inhibitor 310 or growth promotor 320 may be provided on the second tip 31 of the second piston 30 in solid, semi-solid or gelified form, i.e. the second tip 31 is coated with the growth inhibitor 310 or growth promotor 320 or the growth inhibitor 310 or growth promotor 320 is attached to the second tip 31. In particular, the growth inhibitor 310 may be an antibiotic provided in a diffusion disk connected to the second tip 31 of the second piston 30.

The downward movement of the second piston 30 may be performed repeatedly to increase the amount of growth inhibitor 310 or growth promotor 320 gradually in a "bolus per bolus" manner.

As depicted in Fig. 2, the first piston 20 and the second piston 30 are configured such that the second tip 31 of the second piston 30 is able to contact the surface 211 of the first growth medium 210 opposite from the filtering membrane 40 (the upper surface in Fig. 1 and 2). Thus, this surface 211 is accessible from the bore 22 of the first piston 20, and the second piston 30 has sufficient length and dimensions, such that the second tip 31 of the second piston 30 is able to touch this surface 211. Depending on the dimensions and the force of the downward movement, the second tip 31 may produce a groove 212 in the first growth medium 210.

According to an alternative embodiment (not shown), the second piston 30 may be moved towards the second end opening 12 to a third position 103 above the surface 211. Subsequently, for example, a liquid growth inhibitor 310 or liquid growth promotor 320 may be dispensed onto the surface 211, e.g. from a reservoir in the second piston 30 or from a separate source.

Fig. 1 and 2 and 4 show just one second piston 30 arranged in the bore 22 of the first piston 20. However, a plurality of second pistons 30 can be arranged, e.g. parallel to each other along the longitudinal axis L, in the bore 22 of the first piston 20. For example, the same growth inhibitor 310 or growth promotor 320 may be added to the first growth medium 210 by sequentially moving the second pistons 30 gradually increase the amount of growth inhibitor 310 or growth promotor 320. Alternatively, the individual second pistons 30 may contain different kinds of growth inhibitors 310 and/or growth promotors 320, and the first growth medium 210 may be subjected to these substances sequentially.

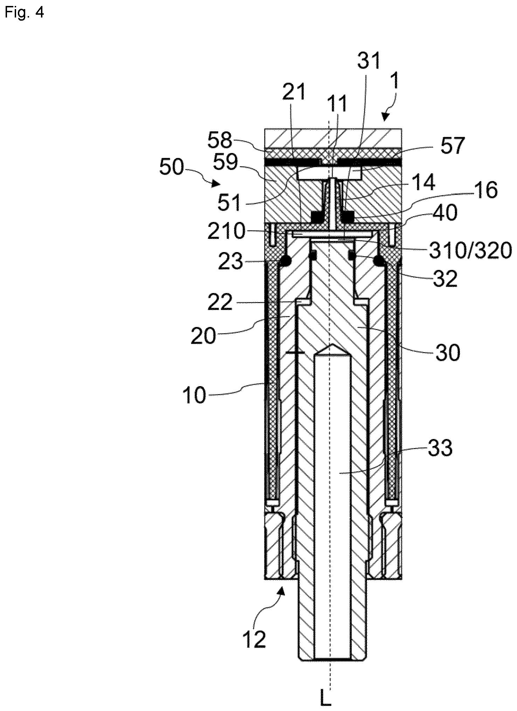

As depicted in Fig. 3, which shows the first tip 21 of the first piston 20 in a cross-sectional view perpendicular to the longitudinal axis L depicted in Fig. 1 and 4, it is also possible within the scope of the invention to provide a plurality of different growth media on the first tip 21 of the first piston 20. This may be achieved by coating a plurality of separate partial areas of the first tip 21 with different growth media (i.e. containing different nutrient compositions, different additives or the like). Fig. 3 shows an example where the tip 21 is divided in eight separate partial areas 201, 202, 203, 204, 205, 206, 207, 208, wherein the first partial area 201 contains the first growth medium 210 and the second partial area 202 contains a second growth medium 220. The other partial areas may contain either the first or the second growth medium, or further different growth media.

It should be noted that all embodiments described above with reference to the first growth medium 210 may equally or analogously applied to the second growth medium 220 and any additional further growth medium. This applies, in particular, to providing growth inhibitors 310 and/or growth promotors 320 to the first growth medium 210 by means of one or several second pistons 30. For example, the first piston 20 with the first tip 21 depicted in Fig. 3 could contain eight or more second pistons 30 in its bore 22 which are configured to supply growth inhibitors 310 or growth promotors 320 to the growth media in the partial areas 201, 202, 203, 204, 205, 206, 207, 208.

Fig. 1 further shows a gas sensor 50 comprising a sensing surface 51 capable of detecting a metabolic gas produced during growth of microorganisms. The gas sensor 50 further comprises a sensor compartment 57, in which the sensing surface 51 is arranged, and which is in fluid connection with the second side 42 of the filtering membrane 40 of the device 1, such that metabolic gases can diffuse from the first side 41 of the filtering membrane 40, where the microorganisms are growing, through the filtering membrane 40 into the sensor compartment 57. In other words, the filtering membrane 40 and the gas sensor 50 have a shared headspace.

Fig. 4 shows a cross-sectional view of a further embodiment of the device 1 comprising analogous components to the device 1 shown in Fig. 1 as well as additional optional details, which are not shown in Fig. 1. The device 1 is depicted in the configuration, where the first growth medium 210 at the first tip 21 of the first piston 20 contacts the first filtering membrane 40, and the growth inhibitor 310 or growth promotor 320 at the second tip 31 of the second piston 30 contacts the first growth medium 210.

According to the embodiment of Fig. 4, the second piston 30 fills substantially the entire bore 22 of the first piston 20.

The first piston 20 according to the embodiment of Fig. 4 comprises a first sealing ring 23 which seals the first piston 20 against the inner wall of the barrel 10. Furthermore, in this embodiment, the second piston 30 comprises a second sealing ring 32 abutting the inner wall of the bore 22 of the first piston 20, thereby sealing the second piston 30 against the first piston 20.

Further, in the embodiment of Fig. 4, the second piston 30 comprises an inner bore 33 which reduces the weight of the second piston 30. This is especially advantageous if the second piston 30 is made from a relatively heavy material, such as a metal.

The gas sensor 50 of the device 1 according to the embodiment shown in Fig. 4 comprises a sensor compartment 57 enclosed by a casing 59. The casing 59 comprises an opening for insertion of the nozzle 14 of the barrel 10. The opening of the casing 59 is connected to the sensor compartment 57, and a third sealing ring 16 on the casing 59 is configured to seal the sensor compartment 57 against the nozzle 14 when the nozzle 14 is inserted into the opening as shown in Fig. 4. A sensing surface 51 of the gas sensor 50 is arranged in the sensor compartment 57 and connected to a printed circuit board 58 comprising electronic components of the gas sensor 50.

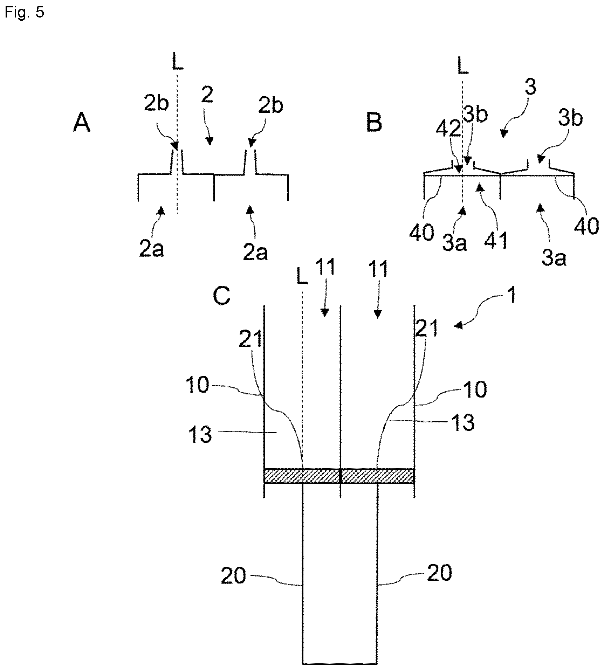

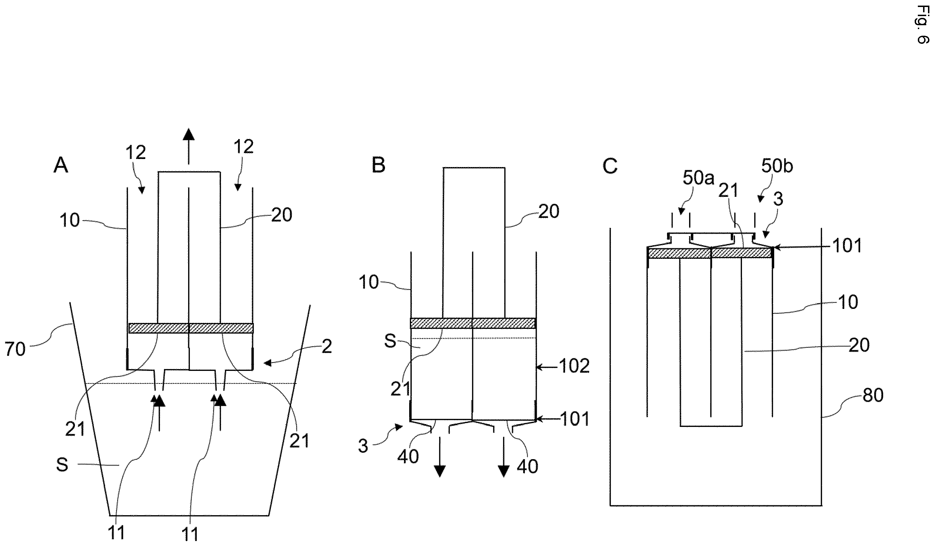

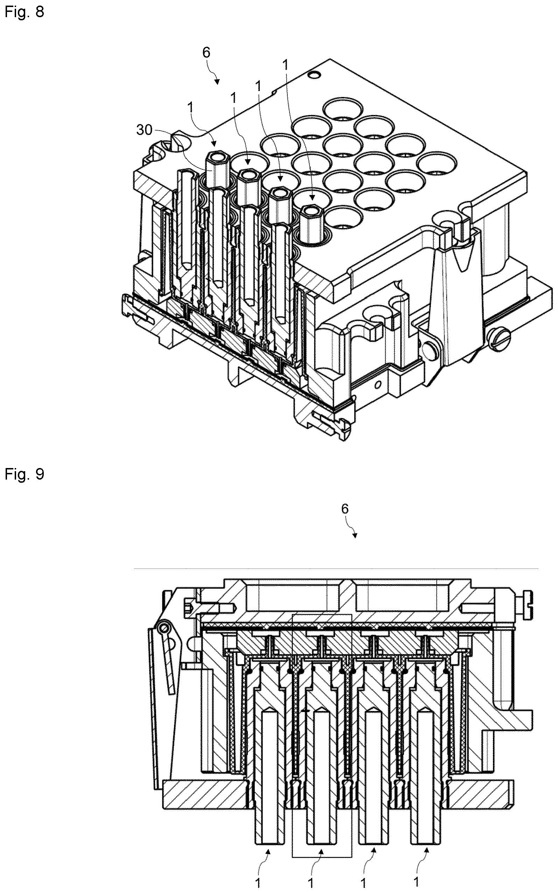

Fig. 5 and 6 show a further embodiment of the device 1, where the filtering membrane 40 is provided in a separate removable filtering cap 3 (Fig. 5 B) and an additional aspiration cap 2 (Fig. 5 A) is provided, e.g. in a kit of parts 5. The depicted embodiment of the device 1 comprises two separate parallel barrels 10 and two associated first pistons 20. However, the illustrated principle may also be equally applied to embodiments with only one barrel 10 and one first piston 20 (e.g. Fig. 1 and 4) or more than two barrels 10 and first pistons 20 (e.g. Fig. 8 and 9).

The aspiration cap 2 shown in Fig. 5A comprises connection openings 2a configured to be releasably connected to the respective first end openings 11 of the device 1 depicted in Fig. 5C. Furthermore, the aspiration cap 2 comprises aspiration openings 2b for aspirating a liquid opposite of the connection openings 2a. The aspiration openings 2b are arranged on nozzles 14.

The filtering cap 3 shown in Fig. 5B comprises connection openings 3a configured to be releasably connected to the respective first end openings 11 of the device 1 depicted in Fig. 5C. Furthermore, the filtering cap 3 comprises dispensing openings 3b opposite the connection openings 3a, and filtering membranes 40 separating the filtering cap 3 in compartments facing the dispensing openings 3b and the connection openings 3a. Therein, the first side 41 of the filtering membranes 40 faces the respective connection opening 3a and the second side 42 of the filtering membranes 40 faces the respective dispensing opening 3b.

Fig. 6 illustrates an embodiment of the method according to the invention using the components shown in Fig. 5A to C. In the step shown in Fig. 6A, the connection openings 2a of the aspirating cap 2 are connected to the respective first end openings 11 of the device 1 (see Fig. 5), and the nozzles 14 comprising the aspiration openings 2b are placed below the surface of a liquid sample S contained in a receptacle 70. Thereafter, the first pistons 20 are moved towards the second end opening 12 of the device 1 (upwards in Fig. 6 A) to aspirate the liquid sample S into the barrel compartments 13 of the barrels 10. When a desired volume of the liquid sample S has been loaded into the barrel compartments 13, the aspiration cap 2 is removed, and a filtering cap 3 (se Fig. 5 B) is connected to the barrel 10 by connecting the connection openings 3a of the filtering cap 3 to the first end openings 11 of the barrel 10.

Subsequently, as depicted in Fig. 6B, the liquid sample S is filtered by the filtering membranes 40 in the filtering cap 3 by moving the first piston 20 towards the first end openings 11 of the barrel 10. Consequently, microorganisms in the liquid sample S are retained on the first side 41 of the filtering membrane 40.

In one embodiment, the first tip 21 of the first piston 20 is advanced to the first position 101, where the first tip 21 contacts the first side 41 of the filtering membrane 40. Thereby, in particular, a solid first growth medium 210 on the first tip 21 (not shown in Fig. 6) may be brought in contact with the filtering membrane 40.

Alternatively, if the first growth medium 210 is a liquid contained in the liquid sample S, the first tip 21 is advanced to the second position 102, where the first tip 21 is spaced apart from the filtering membrane 40. In this manner, a volume of the liquid sample S remains in the barrel compartment 13.

In both cases, the filtered liquid is particularly discarded.

Fig. 6C depicts an incubation and detection step, where the device 1 is placed in an incubator 80 configured to heat the device 1 to a desired incubation temperature suitable to allow growth of the microorganisms of interest, e.g. 37°C. The first end openings 11 of the device 1 are respectively connected to a first gas sensor 50a and a second gas sensor 50b to detect metabolic gases produced by the growing microorganisms on the first side 41 of the filtering membranes 40 and/or in the first growth medium 210. The gas sensors 50a, 50b each have a shared headspace with the respective second side 42 of the respective first filtering membrane 40, such that the metabolic gases diffuse to the respective sensing surface 51 (not shown in Fig. 6, see Fig. 1 and 4) of the respective gas sensor 50a, 50b without the danger of microorganisms contaminating the gas sensors 50a, 50b.

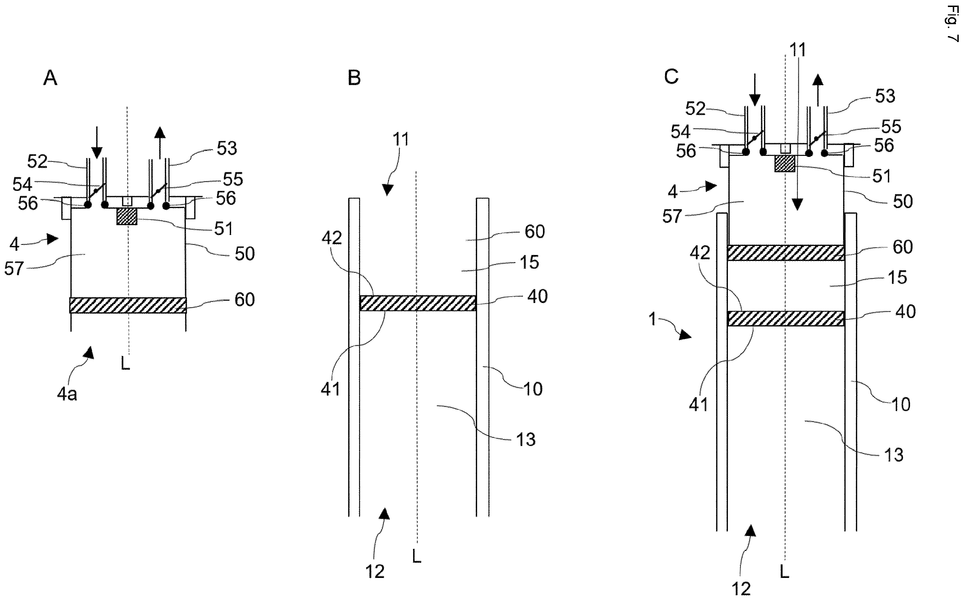

Fig. 7A-C show an embodiment of the device 1 for culturing and detecting microorganisms, where the gas sensor 50 is comprised in a sensor cartridge 4 which is removably connected to the first end opening 11 of the device 1. Fig. 7A shows the disconnected sensor cartridge 4 comprising the connection opening 4a. Fig. 7B schematically depicts the barrel 10 of the device with the first end opening 11 and the second end opening 12 and the first filtering membrane 40. In Fig. 7C, the assembled device 1 is shown, wherein the connection opening 4a of the sensor cartridge 4 has been connected to the first end opening 11 of the barrel 10, e.g. by a plug-in connection. In Fig. 7B and 7C, only the barrel 10 and the first filtering membrane 40 of the device 1 are shown for simplicity. Of course, a first piston 20 and an optional second piston 30 similar to those shown in Fig. 1 and 4 may be provided in the barrel compartment 13 of the device 1 shown in Fig. 7.

The gas sensor 50 comprises a sensor compartment 57, in which a sensing surface 51 is arranged. The sensor compartment 57 is delimited by a second filtering membrane 60, providing an additional barrier against contamination of the sensor compartment 57, the second filtering membrane 60 having a first side facing the sensor compartment 57 and a second side facing the outside of the sensor cartridge 4.

When the sensor cartridge 4 and the device 1 are assembled, an intermediate compartment 15 is formed between the second side 42 of the first filtering membrane 40 and the second side of the second filtering membrane 60.

Metabolic gases produced by microorganisms growing on the first side 41 of the first filtering membrane 40 diffuse through the first filtering membrane 40 into the intermediate compartment 15 and subsequently through the second filtering membrane 60 into the sensor compartment 57, where the metabolic gases can be detected at the sensing surface 51.

In addition, the gas sensor 50 shown in Fig. 7 comprises an inlet 52 and an outlet 53 connecting the sensor compartment 57 to the exterior of the gas sensor 50. The inlet 52 comprises a first valve 54, e.g. a check valve, allowing gas flow into the sensor compartment 57, and the outlet 53 comprises a second valve 55, e.g. a check valve, allowing gas flow out of the sensor compartment 57. Seals 56, such as sealing rings, are arranged at the interface of the inlet 52 and the wall delimiting the sensor compartment 57 and at the interface of the outlet 53 and the wall delimiting the sensor compartment 57, providing a gas-tight seal.

The sensor compartment 57 may be flushed with a desired flushing gas through the inlet 52 and the outlet 53. For example, before usage of the gas sensor 50, one or more calibrant gases may be fed into the inlet 52 and removed from the outlet 53 of the sensor compartment 57 as a steady state flow. Furthermore, in the idle state of the gas sensor 50, the sensor compartment 57 may be flushed with an air flow, e.g. at 95% relative humidity and the desired culturing temperature (e.g. 37°C). For instance, the air flow may consist of 'zero air' or at least a N₂/O₂ mixture or ambient air, from which volatile organic compounds have been removed. In particular, the term 'zero air' as used herein refers to air with a concentration of 0,05 ppm or less of organic compounds. Thereby, the baseline of the gas sensor 50 may be adjusted before it is exposed to the growing culture of microorganisms. This improves sensitivity.

The above-described functionality may also be embodied in a gas sensor 50 which is not comprised in a removable sensor cartridge 4. That is, such a gas sensor 50 may comprise an inlet 52 comprising a first valve 54, e.g. a check valve, allowing gas flow into the sensor compartment 57, and an outlet 53 comprising a second valve 55, e.g. a check valve, allowing gas flow out of the sensor compartment 57.

Fig. 8 and 9 show different views of an apparatus 6 comprising a plurality of devices 1 arranged in parallel to each other to form a two-dimensional array. In particular, the devices 1 are formed and configured as the device 1 shown in Fig. 4. In particular, each device comprises a barrel 10, a first piston 20, a second piston 30, a first filtering membrane 40 and a gas sensor 50 in fluid connection with the second side 42 of the respective first filtering membrane 40. Fig. 8 shows a perspective, cut-away view of the apparatus 6 and Fig. 9 is a cross-sectional view through one row of four devices 1. Such an apparatus 6 has the advantage that several liquid samples S may be tested for microorganisms in parallel. Using the apparatus 6, it is also possible to test microorganisms in a liquid sample S for resistance and susceptibility to a number of different growth inhibitors, such as antibiotics.

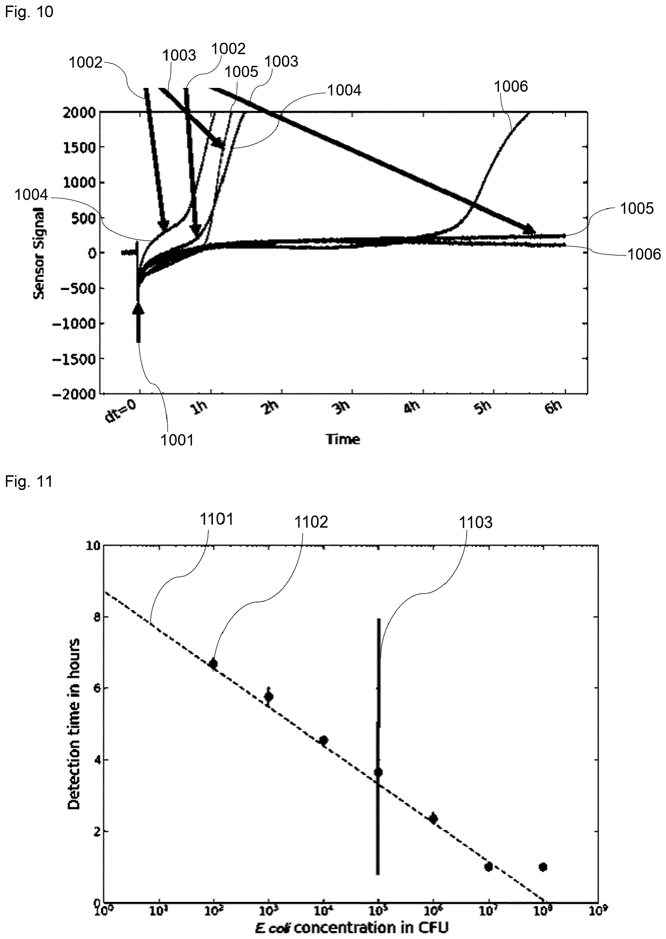

Fig. 10 and 11 show data obtained with a device 1 according to the invention. The plots shown in Fig. 10 were obtained by culturing microorganisms from three different patient samples suffering from a urinary tract infection: the analyzed urine sample of patient 1 had a high bacterial load of bacteria resistant to a certain antibiotic (trace 1004), the urine sample from patient 2 had a high bacterial load of bacteria susceptible to the antibiotic (trace 1005), and the urine sample of patient 3 had a low bacterial load of bacteria susceptible to the antibiotic (trace 1006).

The start of the experiment is indicated by the arrow 1001. Arrows 1002 indicate cultures without addition of the antibiotic, whereas arrows 1003 indicate cultures to which the antibiotic was added at the start 1001 of the experiment. The x-axis of the plot in Fig. 10 shows time in hours after the start 1001 of the experiment and the y-axis displays the signal of the gas sensor 50 of the device 1 in arbitrary units (a.u.).

It can be observed from Fig. 10 that without antibiotics in case of the resistant high bacterial load sample 1004 and the susceptible high bacterial load sample 1005, the amount of metabolic gases detected by the gas sensor 50 sharply rises to 2000 a.u. after about 1 hour from the start 1001 of the experiment. The low bacterial load susceptible sample 1006 gives rise to the same gas sensor 50 signal after about 5,5 h.

A marked difference is observed in the presence of antibiotics. Whereas the resistant high bacterial load sample 1004 displays a sharp increase of the gas sensor 50 signal lagging behind the signal without antibiotics by about 0,5 h, no increase of the gas sensor 50 signal is observed for the high bacterial load susceptible sample 1005 and the low bacterial load susceptible sample 1006, which remain slightly above baseline after 6 h.

This shows that the response of bacterial cultures to antibiotics can be accurately traced by the device 1 according to the invention.

Fig. 11 shows a plot of *E.coli* concentration in colony forming units from automatic growth detection 1102 against detection time in hours. The indicated colony forming units have been determined at the start of the experiment. An exponential fit 1101 and the WHO infection border 1103 are indicated. For example, automatic growth detection may be performed by analyzing the relative level, the slope and/or the curvature of the traces shown in Fig. 10 after normalization of the data against the baseline.

**List of reference numerals**

| | |
|---|---|
| 1 | Device for culturing and detecting microorganisms |
| 2 | Aspiration cap |
| 2a | Connection opening of aspiration cap |
| 2b | Aspiration opening |
| 3 | Filtering cap |
| 3a | Connection opening of filtering cap |
| 3b | Dispensing opening |
| 4 | Sensor cartridge |
| 4a | Connection opening of sensor cartridge |
| 5 | Kit of parts |
| 6 | Apparatus |
| 10 | Barrel |
| 11 | First end opening |
| 12 | Second end opening |
| 13 | Barrel compartment |
| 14 | Nozzle |
| 15 | Intermediate compartment |
| 16 | Third sealing ring |
| 20 | First piston |
| 21 | First tip |
| 22 | Bore |
| 23 | First sealing ring |
| 30 | Second piston |
| 31 | Second tip |
| 32 | Second sealing ring |
| 33 | Bore |
| 40 | First filtering membrane |
| 41 | First side of first filtering membrane |
| 42 | Second side of first filtering membrane |
| 50 | Gas sensor |
| 50a | First gas sensor |
| 50b | Second gas sensor |
| 51 | Sensing surface |
| 52 | Inlet |
| 53 | Outlet |
| 54 | First valve |
| 55 | Second valve |
| 56 | Seal |
| 57 | Sensor compartment |
| 58 | Printed circuit board |
| 59 | Casing |
| 60 | Second filtering membrane |
| 70 | Receptacle |
| 80 | Incubator |
| 101 | First position |
| 102 | Second position |
| 103 | Third position |
| 201 | First partial area |
| 202 | Second partial area |
| 203, 204, 205, 206, 207, 208 | Further partial areas |
| 210 | First growth medium |
| 211 | Surface of first growth medium |
| 212 | Opening |
| 310 | Growth inhibitor |
| 320 | Growth promotor |
| 1001 | Start of measurement |
| 1002 | Measurement without growth inhibitor |
| 1003 | Measurement with growth inhibitor |
| 1004 | Resistant microorganisms-high bacterial load |
| 1005 | Susceptible microorganisms-high bacterial load |
| 1006 | Susceptible microorganisms - low bacterial load |
| 1101 | Exponential fit |
| 1102 | Automatic growth detection |
| 1103 | WHO infection border |
| L | Longitudinal axis |
| S | Liquid sample |

## Claims

1. A method for culturing and detecting microorganisms, comprising the steps of
a. providing a liquid sample (S) in a barrel (10) of a device (1) for culturing and detecting microorganisms, wherein said liquid sample (S) is particularly dispensed into said barrel (10) or aspirated into said barrel (10) via a first end opening (11) of said barrel (10),
b. passing the liquid sample (S) through a first filtering membrane (40) from a first side (41) to a second side (42) of the first filtering membrane (40), the second side (42) being arranged opposite the first side (41), such that microorganisms contained in the liquid sample (S) are retained at the first side (41) of the first filtering membrane (40),
c. contacting said first side (41) of the first filtering membrane (40) with a first growth medium (210) capable of supporting growth of microorganisms,
d. incubating the first filtering membrane (40) and the first growth medium (210) at an incubation temperature,
e. arranging a sensing surface (51) of a gas sensor (50) in fluid connection with the second side (42) of the first filtering membrane (40),
f. detecting a metabolic gas released by the microorganisms by means of the gas sensor (50).

2. The method according to claim 1, wherein said first growth medium (210) is a solid, a gelified liquid or a liquid adsorbed into a porous solid or semi-solid, and wherein said device comprises a first piston (20) comprising a first tip (21), and wherein said first tip (21) of the first piston (20) comprises said first growth medium (210), wherein said first side (41) of said first filtering membrane (40) is contacted by said first growth medium (210) by moving said first piston (20) in said barrel (10) to a first position (101) along a longitudinal axis (L) of the barrel (10).

3. The method according to claim 2, wherein said first tip (21) of said first piston (20) comprises a first partial area (201) and a second partial area (202), the first partial area (201) and the second partial area (202) extending perpendicular to the longitudinal axis (L), wherein the first partial area (201) and the second partial area (202) are non-overlapping, and wherein said first growth medium (210) is arranged in or on the first partial area (201), and wherein a second growth medium (220) is arranged in or on the second partial area (202).

4. The method according to claim 1, wherein said first growth medium (210) is a liquid comprised in said liquid sample (S), and wherein the first side (41) of said first filtering membrane (40) is contacted by the first growth medium (210) upon providing said liquid sample (S) in said barrel (10) or by dispensing the liquid sample (S) through the first filtering membrane (40), wherein said liquid sample (S) is dispensed through the first filtering membrane (40) by moving the first piston (20) in the barrel (10) along the longitudinal axis (L) towards the filtering membrane (40) to a second position (102), wherein said first tip (21) of said first piston (20) is spaced apart from said first filtering membrane (40) in the second position (102), such that a volume of said liquid sample (S) comprising the liquid first growth medium (210) remains in the barrel (10) and contacts said first side (41) of said first filtering membrane (40).

5. The method according to any one of the preceding claims, wherein said first growth medium (210) is contacted by a growth inhibitor (310) capable of inhibiting growth of microorganisms or a growth promotor (320) capable of promoting growth of microorganisms.

6. The method according to claim 5, wherein said growth inhibitor (310) or said growth promotor (320) is comprised in or on a second tip (31) of a second piston (30) of the device, and wherein said first growth medium (210) is contacted by said growth inhibitor (310) or said growth promotor (320) by moving said second piston (30) along said longitudinal axis (L) to a third position (103) in a bore (22) of said first piston (20).

7. The method according to any one of the preceding claims, wherein the liquid sample (S) is incubated with magnetic beads carrying an affinity molecule on their surface, the affinity molecule being capable of specifically binding to microorganisms, wherein a suspension of the magnetic beads that have been incubated with the liquid sample (S) is provided in the barrel (10) of the device (1) for culturing and detecting microorganisms, wherein the first filtering membrane (40) comprises or consists of a magnetic material providing a magnetic force, and wherein the magnetic beads are precipitated on the first side (41) of the first filtering membrane (40) by the magnetic force.

8. A device (1) for culturing and detecting microorganisms, comprising
a. a barrel (10) extending along a longitudinal axis (L) between a first end opening (11) and a second end opening (12), the barrel (10) enclosing a barrel compartment (13) for receiving a liquid sample (S),
b. a first piston (20) comprising a first tip (21), wherein said first piston (20) is movable in said barrel (10) along the longitudinal axis (L) of the barrel (10),
**characterized in that**
said barrel compartment (13) is configured to be brought in fluid communication via a first filtering membrane (40) with a sensing surface (51) of a gas sensor (50) configured to detect a metabolic gas released by microorganisms, wherein the first filtering membrane (40) comprises a first side (41) and a second side (42) opposite the first side (41), wherein the first side (41) of the first filtering membrane (40) faces the barrel compartment (13) and the second side (42) of the first filtering membrane (40) faces the sensing surface (51), and wherein the first filtering membrane (40) is configured to retain microorganisms contained in the liquid sample (S) at the first side (41) of the first filtering membrane (40), particularly wherein the device (1) comprises said first filtering membrane (40) and/or said gas sensor (50).

9. The device (1) according to claim 8, **characterized in that** the first tip (21) of the first piston (20) comprises a first growth medium (210) capable of supporting growth of microorganisms, wherein said first piston (20) is movable along the longitudinal axis (L) in the barrel (10) to a first position (101), in which the growth medium (210) contacts the first side (41) of the first filtering membrane (40).

10. The device (1) according to claim 9, **characterized in that** said first tip (21) of said first piston (20) comprises a first partial area (201) and a second partial area (202), the first partial area (201) and the second partial area (202) extending perpendicular to the longitudinal axis (L), wherein the first partial area (201) and the second partial area (202) are non-overlapping, and wherein said first growth medium (210) is comprised in or on the first partial area (201), and wherein a second growth medium (220) is comprised in or on the second partial area (202).

11. The device (1) according claim 9 or 10, **characterized in that** the device (1) comprises at least one second piston (30) comprising a second tip (31), wherein said second piston (30) is movable along the longitudinal axis (L) in the barrel (10), particularly in a bore (22) of the first piston (20), to a third position (103), wherein the second tip (31) comprises or is configured to comprise a growth inhibitor (310) capable of inhibiting growth of microorganisms or a growth promotor (320) capable of promoting growth of microorganisms.

12. The device (1) according to any one of the claims 8 to 11, **characterized in that** the sensing surface (51) of said gas sensor (50) is arranged in a sensor compartment (57) which is in fluid connection with the second side (42) of the first filtering membrane (40), wherein said sensor compartment (57) comprises an inlet (52) and an outlet (53), wherein said inlet (52) comprises a first valve (54) allowing gas flow into the sensor compartment (57), and wherein the outlet (53) comprises a second valve (55) allowing gas flow out of the sensor compartment (57).

13. The device (1) according to any one of the claims 8 to 12, **characterized in that** the first filtering membrane (40) comprises or consists of a magnetic material providing a magnetic force, such that magnetic beads comprised in the liquid sample (S) can be precipitated on the first side (41) of the first filtering membrane (40) by the magnetic force.

14. A sensor cartridge (4) comprising a gas sensor (50) comprising a sensing surface (51) configured to detect a metabolic gas released by microorganisms, wherein the sensor cartridge (4) comprises a connection opening (4a) configured to be connected to the first end opening (11) of the barrel (10) of the device (1) according to any one of the claims 8 to 13.

15. A kit of parts (5) comprising
a. the device (1) according to one of the claims 8 to 13,
b. a filtering cap (3) extending along a longitudinal axis (L) between a connection opening (3a) and a dispensing opening (3b), wherein the filtering cap (3) comprises a first filtering membrane (40) comprising a first side (40) facing the connection opening (3a) and a second side (42) opposite the first side (41), the second side (42) facing the dispensing opening (3b), wherein the filtering cap (3) is configured to be removably connected to the first end opening (11) of the barrel (10) of the device (1), such that a liquid sample (S) can be passed through the first filtering membrane (40) from the first side (41) to the second side (42) of the first filtering membrane (40) to retain microorganisms contained in the liquid sample (S) at the first side (41) of the first filtering membrane, wherein the dispensing opening (3b) of the filtering cap (3) is configured to be brought in fluid communication with a sensing surface (51) of a gas sensor (50) configured to detect a metabolic gas released by microorganisms, and
c. a sensor cartridge (4) comprising a gas sensor (50) comprising a sensing surface (51) configured to detect a metabolic gas released by microorganisms, wherein the sensor cartridge (4) comprises a connection opening (4a) configured to be connected to the connection opening (3a) of the filtering cap (3),
d. optionally, an aspiration cap (2) extending along a longitudinal axis (L) between a connection opening (2a) and an aspiration opening (2b) opposite the connection opening (2a), wherein the aspiration cap (2) is configured to be removably connected to the first end opening (11) of the barrel (10) of the device, such that a liquid sample (S) can be aspirated through the aspiration opening (2b) into the barrel compartment (13) of the device (1) when the first piston (20) of the device (1) is moved along the longitudinal axis (L) in the direction from the first end opening (11) towards the second end opening (12).
